(19) Europäisches Patentamt
European Patent Office
Office européen des brevets

(11) Publication number: **0 147 704**
**B1**

(12) # EUROPEAN PATENT SPECIFICATION

(45) Date of publication of patent specification: **19.04.89**

(51) Int. Cl.[4]: **C 10 L 1/22,** G 01 N 33/28

(21) Application number: **84115025.3**

(22) Date of filing: **10.12.84**

(54) **A water immiscible liquid comprising a tagging compound and method for detecting such tagging compound.**

(30) Priority: **16.12.83 US 562331**

(43) Date of publication of application:
**10.07.85 Bulletin 85/28**

(45) Publication of the grant of the patent:
**19.04.89 Bulletin 89/16**

(84) Designated Contracting States:
**BE DE FR GB IT NL**

(56) References cited:
**CA-A-1 016 941**
**FR-A-1 306 040**
**FR-A-2 322 174**
**US-A-1 898 953**
**US-A-3 164 449**
**US-A-3 883 568**

(73) Proprietor: **MORTON THIOKOL, INC.**
**110 North Wacker Drive**
**Chicago Illinois 60606-1560 (US)**

(72) Inventor: **Orelup, Richard B.**
**19 Pembroke Trial**
**Upper Saddle River New Jersey 07458 (US)**

(74) Representative: **Dr. Elisabeth Jung Dr. Jürgen Schirdewahn Dipl.-Ing. Claus Gernhardt**
**P.O. Box 40 14 68 Clemensstrasse 30**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 147 704 B1**

Courier Press, Leamington Spa, England.

**Description**

The invention relates to tagged liquids comprising compounds suitable for marking or dyeing organic liquids, particularly petroleum based fuels and to a method for detecting such compounds.

Background Art

A dye is defined herein as a material lending visible colour when dissolved in the dyed product. Examples of dyes which have been used for dyeing organic liquids are Color Index Solvent Red #24, Solvent Red #19, Solvent Yellow #14, Solvent Blue #36, and Solvent Green #3.

A marker is defined herein as a substance which can be dissolved in a liquid to be identified then subsequently detected by performing a simple chemical or physical test on the marked liquid. Markers that have been proposed, or are in use, include furfural, quinizarin, diphenylamine and radioactive materials. (Radioactive materials have not been accepted in Western countries because of special equipment and precautionary measures associated with their handling.)

Dyes and markers (referred to collectively as "tags" herein) are needed to clearly distinguish chemically or physically similar liquids. As one example, fuels are dyed to provide visually distinctive brand and grade denominations for commercial and safety reasons. As another example, some lightly taxed products are tagged to distinguish them from similar materials subject to higher taxes. Furthermore, certain fuels are dyed or marked to deter fraudulent adulteration of premium grade products with lower grade products, such as by blending kerosene, stove oil, or diesel fuel into regular grade gasoline or blending regular grade gasoline into premium grade gasoline. Identification of particular batches of bulk liquids for protection against theft is another valuable function of markers and dyes, particularly for identifying fuels owned by large government, military or commercial consumers. Finally, marketers of brand name products tag their products with dyes or markers to detect substitution of others' products in their distribution system.

Dyes alone are not always adequate to securely and reliably tag liquids. Many dyes are easily removed by unauthorized persons. Furthermore, dyes can be obscured by other natural or added substances (particularly dyes present at low concentrations in a mixture of fuels). Because dyes alone have these shortcomings, a combination of a dye and a marker often is used to tag an organic liquid.

Many commonly used markers are also less than ideal for tagging bulk liquids. Quinizarin and diphenylamine both are fairly sensitive marking materials, with simple detection procedures, but have the disadvantage of poor solubility in nonopolar materials. Their solubility in commonly used petroleum solvents is less than 1%, meaning that an undesirably large volume of a concentrated marker solution must be transported and handled to mark a given volume of fuel. (To encourage rapid and complete dissolution of the marker, it is usually added to the liquid to be marked in the form of a concentrated solution, rather than as a pure compound.)

Furfural has previously been used as a marker for middle distillate fuels. It is extracted by a 10% solution of aniline in acetic acid to form a strongly colored bluish red complex in the lower reagent layer. While quite sensitive, this test has serious disadvantages. First, the slightest contamination of the fuel by residual furfural (which is sometimes used in refining) gives a false positive test. Second, furfural is unstable in certain oils and may not be detectable in such oils after the usual storage period of three to six months. Third, middle distillate fuels tend to discolor appreciably during storage. Some of this discoloration is extracted by aniline acetate and can partially or totally obscure a positive furfural test, particularly if the furfural marked fuel is used to adulterate more expensive or highly taxed unmarked fuel.

As a specific example, furfural has been used in several European countries as a marker for fuel oil at concentrations of 5—10 parts per million (ppm). While certain relatively clean oils give a positive test for as little as 0.5 ppm of furfural, lower quality oils have been observed to give no distinguishable test at concentrations of up to 2 ppm. For such oils the furfural marker cannot be detected in mixtures containing 20%—40% marked fuel. Thus, the less expensive marked fuel can be used in substantial quantities as a diluent without being detected.

Furfural marked fuels have commonly been dyed red, particularly using Solvent Red #19, Solvent Red #24, or related derivatives. But the discoloration previously referred to also obscures the dye, thus preventing visual confirmation that the fuel is tagged.

Recent changes in refining practices further threaten the utility of furfural/red dye combination tagging. In the fairly clean and stable straight run fuels prevalent until recently, furfural is an acceptable marker. But a combination of higher oil prices and sharply increasing demand for diesel fuel as a proportion of the total motive fuel market has necessitated the increasing use of severely cracked heavier grades of crude oil. Severely cracked oils tend to be somewhat unstable, are relatively highly colored when fresh, and tend to destroy the furfural used to mark them. Discoloration and the tendency to destroy the furfural marker both increase as these oils are aged. Tests have shown that 30% to 40% of the furfural is decomposed when it is stored four days in such fuels.

While current practice in many European refineries is production of straight run and cracked fuel oil blends containing 5%—15% cracked product, which will coexist marginally with its furfural marker, it is estimated that the proportion of heavily cracked fuel will exceed 30% in a few years. Under these conditions furfural will be of little or no value as a marker.

The patent literature discloses compounds which are structurally related to the materials disclosed

herein as tagging compounds for fuels or other organic liquids. Many of the compounds disclosed herein are novel. The compounds disclosed here which are not novel *per se* have not been disclosed in the prior art to be useful as a combined dye and marker.

U.S. Patent Nos. 3,764,273 and 3,883,568 disclose 2(2-ethylhexyl)-1,4-dihydroxyanthraquinone:

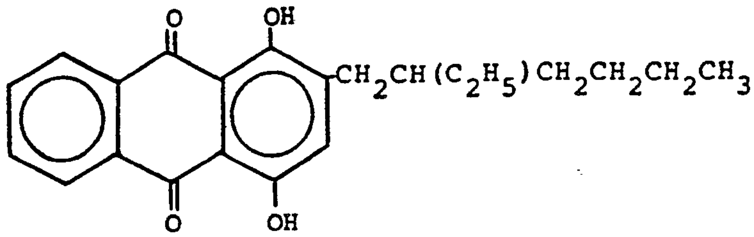

and other substituted anthraquinone in which the depending alkyl moiety has from 1 to 20 carbon atoms, and describes them as being useful as markers for water immiscible organic liquids. But these markers are disclosed to be colorless when employed as markers, and thus do not function both as dyes and as markers at a single concentration.

U.S. Patent No. 3,192,117, U.S. Patent No. 3,454,604, U.S. Patent No. 3,793,349 and FR—A—2,322,174 respectively disclose the following compounds:

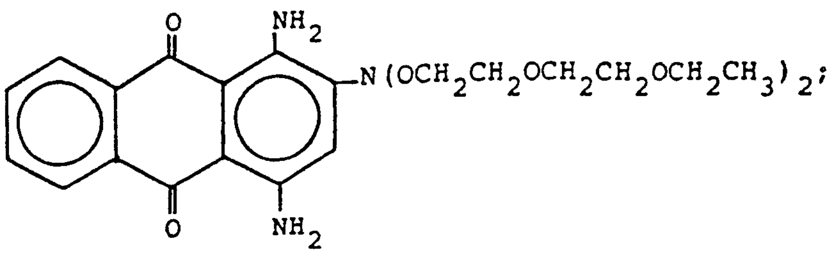

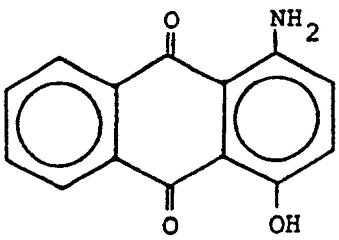 ; 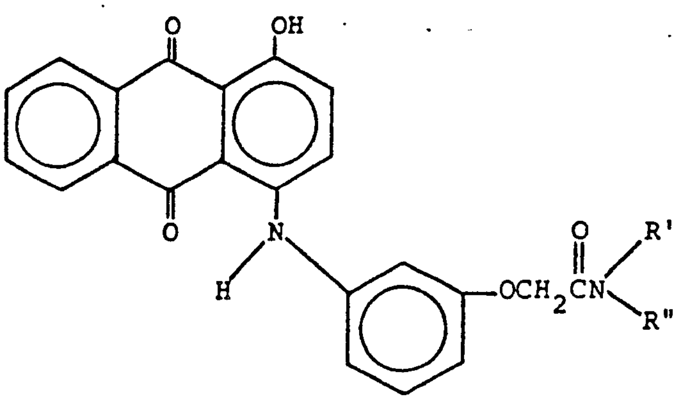 ; and

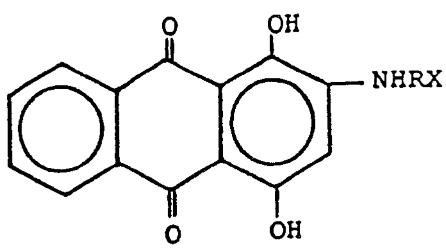

(In the latter compound, R is selected from propyl, isopropyl, or ethyl and X is selected from chloride, bromide, cyanide, or 1 to 4 carbon alkoxy. Specific examples of the amino substituent disclosed in the FR—A—publication include the following:

—$NHCH_2CH_2OCH_3$;
—$NHCH_2CH_2CH_2OCH_3$;
—$NHCH_2CH(CH_3)OCH_3$;
—$NHCH_2CH_2OCH_2CH_3$;

$-NHCH_2CH_2CH_2OC_2H_5$.

The compounds disclosed in the four previously listed patents are respectively disclosed to have utility as hair dyes; dyestuffs; blue dyes for polyester fibers; and pigments for transfer printing on polyesters, polyacrylonitriles or paper. None of these compounds are disclosed to be useful as markers or dyes for organic liquids.

U.S. Patent No. 3,435,054, U.S. Patent No. 3,164,449 and British Specification 452,421 disclose structurally related dyes. The dyes disclosed in the U.S. patents are for fuels, and those in the British specification are for cellulose esters and ethers.

Matsuoka, et al., "A Novel 2-Amination of Quinizarin Promoted by Copper Ions," *Dyes and Pigments* 1:27—37 (1980), teaches synthesis of various 2-amino-1,4-dihydroxyanthraquinones.

CA—A—1 016 941 discloses 2-(substituted)amino-1,4-hydroxyanthraquinone compounds in which the substituent is a straight or branched aliphatic chain having from 4 to 18 carbon atoms. Such compounds are dyes that can be used in hydrocarbon liquids like fuels or gasolines. They do, however, not perform the double function as dyes and markers.

Summary of the Invention

The objects fulfilled by this invention are to provide tagged organic liquids comprising tagging compounds suitable for use with petroleum fuels and other organic liquids, the tagging compounds having the following properties:

1. they are entirely foreign to the liquids;
2. they can be supplied as highly concentrated solutions in compatible solvents.
3. they are easily detected by a simple field test;
4. they are not obscured by unstable natural components of the liquids;
5. they are stable over the anticipated storage life of the tagged liquid (usually three to six months);
6. they have identitites which can be confirmed by laboratory methods; and
7. they act as both a marker and a dye by simultaneously imparting an acceptably intense red color and functioning as a marker at low concentration in the tagged liquid.

These objects are realized by a water-immiscible tagged organic liquid comprising from 1 ppm to 300,000 ppm of a tagging compound having the structure selected from:

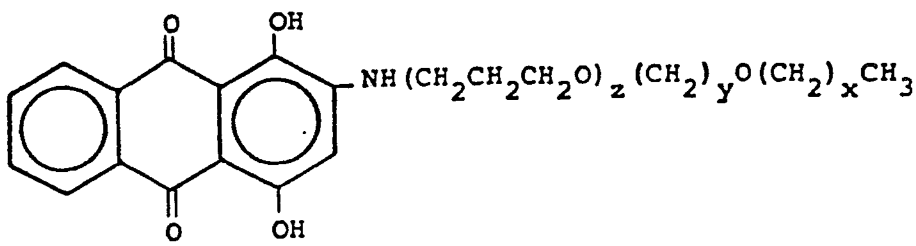

wherein x is an integer between 0 and 3 inclusive, y is an integer between 1 and 3 inclusive, and z is an integer between 0 and 1 inclusive.

Tagging compounds of the above formula in which z is the integer 1 are claimed in co-pending application 84115024.6 (EP—A—149 125) having the identical filing date for which the identical US-priority has been claimed.

Detailed Description of the Invention

One preferred category of tagging compounds used in the water-immiscible tagged organic liquids according to the invention is that of substituted 1,4-hydroxyanthraquinones having the structure shown previously, wherein z is one. Specific examples of such compounds which are believed to be novel are:

(1) 2-(methoxyethoxypropylamino)-1,4-dihydroxyanthraquinone; and

(2) 2-(ethoxyethoxypropylamino)-1,4-dihydroxyanthraquinone.

Another preferred category of such compounds is that wherein z is zero. Specific examples of such compounds which are believed to be novel are:

(3) 2-(butoxypropylamino)-1,4-dihydroxyanthraquinone; and

(4) 2-(propoxypropylamino)-1,4-dihydroxyanthraquinone.

Examples of such compounds whose structure is disclosed in the previously-discussed FR—A—publication but whose utilities as tagging compounds combining the properties of a fuel dye and marker are not believed to be known, are:

(5) 2-(methoxypropylamino)-1,4-dihydroxyanthraquinone;

(6) 2-(methoxyethylamino)-1,4-dihydroxyanthraquinone; and

(7) 2-(ethoxyethylamino)-1,4-dihydroxyanthraquinone.

Further examples of such compounds, also believed to be novel, are as follows:

(8) 2-(methoxypropoxypropylamino)-1,4-dihydroxyanthraquinone;

(9) 2-(ethoxymethylamino)-1,4-dihydroxyanthraquinone;

(10) 2-(ethoxymethoxypropylamino)-1,4-dihydroxyanthraquinone;
(11) 2-(ethoxypropylamino)-1,4-dihydroxyanthraquinone;
(12) 2-(propoxyethylamino)-1,4-dihydroxyanthraquinone;
(13) 2-(butoxyethylamino)-1,4-dihydroxyanthraquinone;

The water-immiscible tagged organic liquids according to the invention may additionally comprise another type of dyes having the general formula

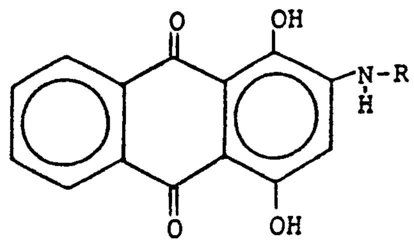

wherein R is selected from linear or branched hydrocarbons having from 1 to 5 carbon atoms.

Specific examples of compounds in which the R substituent is a straight or branched chain hydrocarbon are as follows:

(14) 2-(methylamino)-1,4-dihydroxyanthraquinone;
(15) 2-(ethylamino)-1,4-dihydroxyanthraquinone;
(16) 2-(ethylamino)-1,4-dihydroxyanthraquinone;
(17) 2-(n-butylamino)-1,4-dihydroxyanthraquinone;
(18) 2-(n-pentylamino)-1,4-dihydroxyanthraquinone;
(19) 2-(2-propylamino)-1,4-dihydroxyanthraquinone;
(20) 2-(2-butylamino)-1,4-dihydroxyanthraquinone; and
(21) 2-(t-butylamino)-1,4-dihydroxyanthraquinone.

One of ordinary skill in the art, using the preceding generic disclosure and specific examples as a guide, can determine other species within the scope of the present invention.

The dual function tags generally are crystalline solids in pure form, but may be conveniently supplied as concentrates containing 10%—30% of the pure compound dissolved in one or more high boiling aromatic solvents or in mixtures of high boiling aromatic solvents with co-solvents such as alkyl phenols. The solvent selected also is not critical, provided that it is sufficiently soluble in the tagged fuel to permit a desirably large final proportion of the tagging compound to be incorporated in the fuel, and provided that the solvent does not interfere with storage or use of the tagged fuel and detection of the tagging compound. Specific examples of high-boiling aromatic solvents useful herein are SURESOL #190, primarily comprising a mixture of methyl naphthalenes, available from Koch Refining Co., Corpus Christi, Texas; and commercial grade xylene (mixed isomers). Specific examples of alkyl phenols useful herein are commercial xylenols (which typically contain some phenols and cresols); *ortho* -sec-butyl phenol; and *para*-nonyl phenol. The fuel to be tagged can also be used as a solvent.

The compounds are typically added to liquids to be tagged at a concentration of 10—15 parts per million of the pure compound, or from about 40 ppm to about 100 ppm of the concentrate. Such concentrations provide a color intensity comparable to that commonly required of dyed fuels, However, fuel's containing the minimum detectable and visible concentration of the pure tagging compound (less than 1 ppm) or a larger concentration of the tagging compound are also within the scope of the invention. The amount of tagging compound is not critical, provided that it is dissolved or stably suspended in the solvent and is as concentrated as its solubility permits.

Fuel tagged as disclosed herein can be detected in fuel mixtures containing as little as 5%—10% tagged fuel, whether visually, in field inspection tests, or in the laboratory. Detection is possible even in the presence of 25%—50% highly cracked fuel oils or gasolines, and the novel marker dyes taught herein are found to be essentially unchanged after at least three months' storage in the same fuels.

Field Procedure for Detecting Tagging Compound

A convenient sample (20—50 ml) of fuel suspected to contain tagged fuel is shaken or otherwise contacted with approximately 1/10 its volume (2 to 5 ml) of an alkaline, fuel immiscible reagent in an appropriately narrow glass vial or bottle of 30—120 ml capacity, then allowed to stand. The reagent comprises from about 20% to about 60% glycerine, from about 20% to about 40% water, and from about 10% to about 50% of an organic amine. Two preferred reagents for use herein are disclosed in the Examples below. The alkaline, fuel immiscible reagent is specific to these markers, meaning extraction of otherwise interfering alkaline reactive colored fuel components is minimized. The reagent settles to the bottom and is colored bluish red if marked fuel is present. If the sample contains no marker, the reagent layer remains colorless (or very slightly yellow for some fuels). The distinction is very apparent even at low marker levels.

Laboratory Procedure for Detecting Tagging Compound

A semiquantitative laboratory procedure for the determination of tagged fuel content comprises passage of a measured sample (conveniently 25 ml) of suspected tagged fuel through an activated alumina column, to which all natural and added colorants adhere. Following this, selective column washes remove essentially all colored materials except the marker which is finally removed, preferably by using the reagent described for use in the field procedure as eluent, collected and brought to volume. Absorbance of the colored solution is measured instrumentally and related to marker concentration.

Examples

The following examples are provided to describe how to make and use the invention and to demonstrate its utility. The scope of the invention is not limited by these examples, but is defined by the claims following this specification.

Example 1

2-(methoxypropylamino)-1,4-dihydroxyanthraquinone was prepared as follows: 60 grams of 1,4-dihydroxyanthraquinone, 15.5 grams of boric acid, and 89 grams of methoxypropylamine was slurried in 125 ml of water. The slurry was stirred and heated to 65° Celsius, and a strong stream of air was bubbled through it. These conditions were maintained about nine hours, or until thin layer chromatography of the reaction mixture failed to isolate any, 1,4-dihydroxyanthraquinone.

The (now uniform) liquid mass was poured into a solution of 150 ml. concentrated hydrochloric acid and 300 ml. water, while stirring. Crystals were given time to agglomerate, then the crystals were filtered, washed until free of acid, and dried. The melting point of the resulting product was 140 degrees Celsius.

A similar procedure is followed to form 2-(methoxyethoxypropylamino)-1,4-dihydroxyanthraquinone (melting point: 114 degrees Celsius) and the other 2-amino substituted -1,4 dihydroxyanthraquinones disclosed herein from the corresponding amine and 1,4 dihydroxyanthraquinone.

Example 2

30 grams of 2-(methoxyethoxypropylamino)-1,4-dihydroxyanthraquinone were dissolved in a mixture containing 35 grams ortho-sec-butyl phenol and 35 grams of commercial xylene. The resulting clear dark red solution remained pourable, stable and uniform at temperatures down to −18°C for an indefinite period.

Forty grams of this solution were dissolved in 1000 liters of straight run #2 heating oil to form a red solution defined as 100% marked fuel. The color intensity, measured instrumentally at its wavelength of maximum absorption (512 nm), was equal to the color intensity of a solution containing five grams of Solvent Red #19 (Color Index 26050) per 1000 liters of heating oil, measured at the wavelength of maximum absorption (520 nm) for Solvent Red #19.

Example 3

Fifty milliliters of the 100% marked heating oil of Example 2 were shaken in a 120 ml oil sample bottle with 5 ml of a reagent comprising (by weight):

54% Glycerine
26% Water
20% Methoxyethoxypropylamine

After standing a few seconds, the reagent layer sank to the bottom and was observed to be colored intensely red.

A 50 milliliter sample of the same straight run heating oil containing no marker or dye was subjected to the same test. The reagent layer remained colorless or very slightly yellowish.

Example 4

The 100% marked heating oil of Example 2 and an unmarked control were stored together at ambient temperature in the dark for a period of three months. The colorimetric test of Example 2 and the marker field test of Example 3 were repeated, with essentially the same results.

Example 5

A mixture containing 10% by volume marked heating oil according to Example 2 and 90% unmarked straight run #2 motor diesel fuel was tested according to the procedure of Example 3. A red color again was observed in the reagent layer and was distinctly different from the color of the reagent used to test the unmarked fuel.

Example 6

Examples 2—5 were repeated except the fuel consisted of a commercial blend comprising 75% straight run fuel and 25% of a severely cracked fuel.

The visual and spectrophotometric color tests were sometimes modified by employing untagged fuel oil in the reference cell to account for the contribution of the natural oil colors, but the comparative marker field tests were usable without modification. Substantially the same results were observed.

Example 7

7.5 grams of 2-(methoxypropylamine)-1,4-dihydroxyanthraquinone and 9.0 grams of 2-(methoxyethoxypropylamino)-1,4-dihydroxyanthraquinone were dissolved to form a clear red solution in a solvent mixture comprising 41.7 grams p-nonyl phenol and 41.7 grams of mixed xylenes. The solution remained pourable, stable and uniform at temperatures down to −18°C for an indefinite period.

67 grams of this solution were easily dissolved in 1000 liters of straight run #2 heating oil to form a red solution defined as 100% marked heating oil. Its color intensity was equal to that of a solution of 5 grams Solvent Red #19 in 1000 liters of the same oil, each color intensity being measured at the wavelength of maximum absorption.

Example 8—11

Examples 2—5 were repeated using the dye solution of Example 7, with essentially the same results.

Example 12

7.6 grams of 2-(methoxypropylamino)-1,4-dihydroxyanthraquinone and 9.0 grams of 2-(methoxyethoxypropylamino)-1,4-dihydroxyanthraquinone were dissolved in a solvent mixture comprising 41.7 grams of commercially available xylenols and 41.7 grams of commercial xylene to form a clear red solution. 67 grams of this solution were easily dissolved in 1000 liters of regular grade leaded gasoline, with essentially the same result as in Example 6.

Examples 13 and 14

Examples 2 and 3 were repeated, using the tagged gasoline of Example 12 as the 100 per cent unmarked fuel. The results were essentially the same.

Example 15

A mixture containing 10% by volume marked regular gasoline (made according to Example 12) and 90% premium grade leaded gasoline was tested according to the procedure of Example 2. The test for the tagging compound was clearly positive, and the resulting reagent layer was readily distinguishable from the colorless or very slightly yellowish reagent layer which resulted when the same test was performed on completely unmarked premium grade leaded gasoline.

Example 16

25 ml of a commercial blended #2 heating oil comprising 75% straight run fuel and 25% of a severely cracked fraction, and containing 67 parts per million of the red tagging composition defined in Example 7, was gravity percolated through a column of activated alumina which had a 10 millimeter diameter and was 50 millimeters long. The activated alumina was ALCOA Type F—20, chromatographic grade. The column was then washed with a small portion of isooctane to remove adhering heating oil. At this point the column was colored strongly yellow-brown, particularly near the top, while the percolated fuel was clear. (The column color intensity and distribution depends on the natural color content of the fuel sample.) Successive washes of acetone and ethyl alcohol were passed through the column, and eluted the yellow and brown natural colors of the fuel.

The tagging compound remained in the column as a purple zone, concentrated at the column head or somewhat diffused along much of its length, depending on the fuel's composition.

As eluant mixture having the following composition (by weight):

25% Glycerine
35% Water
40% Cyclohexylamine

was gravity percolated through the column. All of the tagging compound could normally be separated using less than 10 ml of the eluant, and the total eluant was diluted to 10 ml in a volumetric flask.

The eluant was subsequently clarified by passing it through an 0.45—0.60 micron particle retention membrane and its absorbance was measured at 556—557 nm. By reference to a calibration curve derived by measuring the absorbance of fuels having known marker concentrations, marked fuel content was determined.

Other primary amines such as n-amylamine, n-butylamine or methoxyethoxypropylamine were substituted in the above reagent without changing the result substantially, although maximum color stability was obtained with cyclohexylamine.

Example 17

A marker composition having the following formulation is prepared;

A. About 30% by weight of an equimolar mixture of 2-(methoxypropylamino)-1,4-hydroxyanthraquinone and 2-(methoxyethoxypropylamino)-1,4-hydroxyanthraquinone;

B. About 35% by weight of high boiling aromatics; and

C. About 35% by weight of ortho-sec-butylphenol.

The exact proportion of component A is adjusted to 40 ppm of the composition dissolved in West German light heating oil has the same color intensity as a solution of 5 ppm of Solvent Red #19, measured

at their respective wavelengths of maximum absorption. The composition is used to mark fuel and is detected as described in Examples 2—6, 15 and 16, with excellent results.

Example 18

Example 17 is repeated, except that component C is para-nonylphenol.

**Claims**

1. A water-immiscible tagged organic liquid comprising from 1 ppm to 300,000 ppm of a tagging compound having the structure selected from:

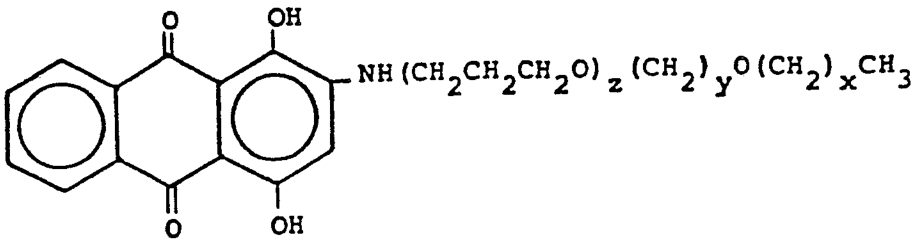

wherein x is an integer between 0 and 3 inclusive, y is an integer between 1 and 3 inclusive, and z is an integer between 0 and 1 inclusive.

2. The tagged liquid of claim 1, comprising from 1 ppm to 15 ppm of said tagging compound.

3. The tagged liquid of claim 1, comprising from 10 ppm to 15 ppm of said tagging compound.

4. The tagged liquid of claim 1 in the form of a concentrate comprising from 100,000 ppm to 300,000 ppm of said tagging compound.

5. The tagged liquid of claim 1, wherein said liquid is a hydrocarbon fuel.

6. The tagged liquid of claims 4 and 5 comprising from 700,000 to 900,000 ppm of a diluent liquid which is soluble in said water-immiscible organic liquid.

7. The tagged liquid of claim 6, wherein said diluent liquid is selected from methyl naphthalenes, xylene, xylenols, ortho-sec-butyl phenol, para-nonyl phenol, and mixtures thereof.

8. A method for forming a liquid concentrate useful for tagging a water-immiscible liquid, comprising the step of blending the following:

A. From 100,000 ppm to 300,000 ppm of a tagging compound having a structure selected from:

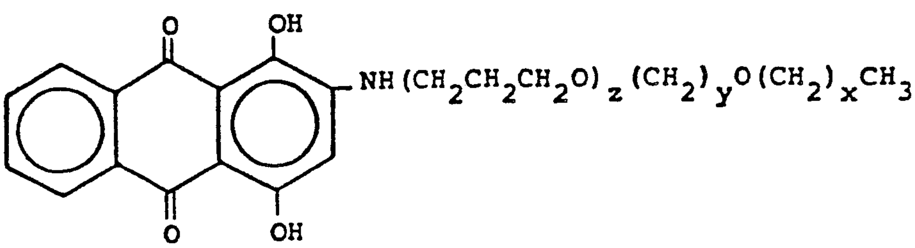

wherein x is an integer between 0 and 3 inclusive, y is an integer between 1 and 3 inclusive, and z is an integer between 0 and 1 inclusive; and

B. from 700,000 to 900,000 ppm of a diluent liquid, which is soluble in said water-immiscible liquid.

9. A method for detecting a tagging compound having a structure selected from:

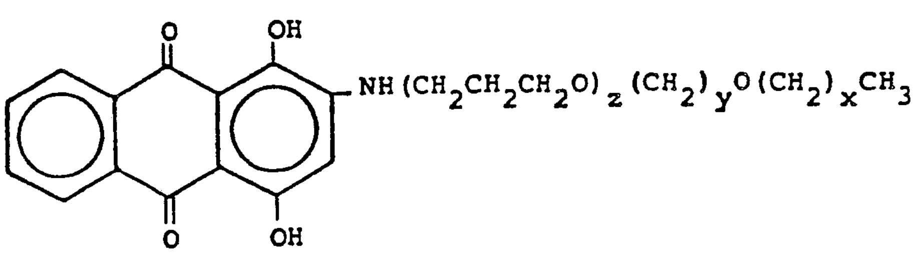

wherein x is an integer between 0 and 3 inclusive, y is an integer between 1 and 3 inclusive, and z is an integer between 0 and 1 inclusive, and

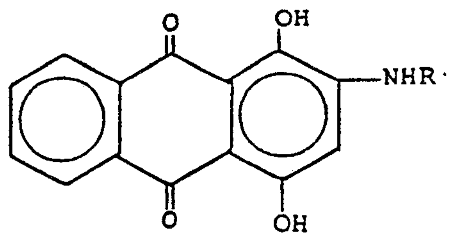

wherein R is selected from linear or branched hydrocarbons having from 1 to 5 carbon atoms, comprising the steps of:

A. providing a sample suspected to contain said tagging compound and

a) carrying out a chemical reaction by contacting said sample

B. with a reagent comprising:

i. from 20 percent to 60 percent glycerine;

ii. from 20 percent to 40 percent water; and

iii. from 10 percent to 50 percent of an organic amine;

C. separating said reagent and said sample; and

D. determining whether said reagent develops a substantially bluish red color, thereby indicating the presence of said tagging compounds, or

b) absorbing said tagging compound and concentrating it by

B. passing said sample through an activated alumina chromatographic column;

C. washing said organic liquid from said column;

D. washing the natural colors of said organic liquid from said column; and

E. eluating with the reagent comprising components i, ii, and iii as described under aB when a substantially purple zone is formed in said column, thereby indicating the presence of said tagging compounds.

**Patentansprüche**

1. Eine mit Wasser nicht mischbare markierte organische Flüssigkeit, umfassend von 1 TpM bis 300 000 TpM einer Markierungsverbindung mit einer Struktur ausgewählt aus:

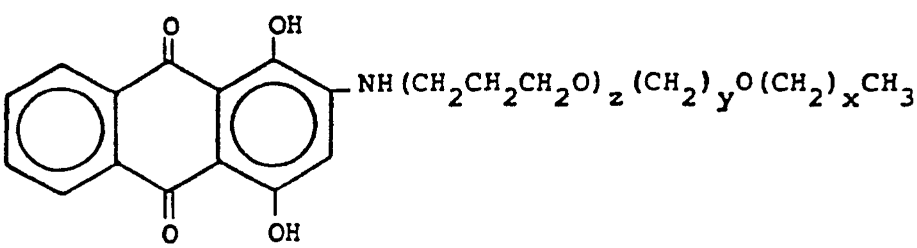

wobei x eine ganze Zahl von 0 bis einschließlich 3 ist, y eine ganze Zahl von 1 bis einschließlich 3 und z eine ganze Zahl von 0 bis einschließlich 1 ist.

2. Die merkierte Flüssigkeit nach Anspruch 1, umfassend von 1 TpM bis 15 TpM besagter Markierungsverbindung.

3. Die markierte Flüssigkeit nach Anspruch 1, umfassend von 10 TpM bis 15 TpM der besagten Markierungsverbindung.

4. Die markierte Flüssigkeit von Anspruch 1 in Form eines Konzentrats, umfassend von 100 000 TpM bis zu 300 000 TpM der besagten Markierungsverbindung.

5. Die markierte Flüssigkeit nach Anspruch 1, in welcher besagte Flüssigkeit ein Kohlenwasserstoffbrennstoff ist.

6. Die markiert Flüssigkeit nach Anspruch 4 und 5 umfassend von 700 000 bis 900 000 TpM einer als Verdünnungsmittel wirkenden Flüssigkeit, welche in der mit Wasser nicht mischbaren organischen Flüssigkeit löslich ist.

7. Die markiert Flüssigkeit nach Anspruch 6, in welcher die als Verdünngsmittel wirkende Flüssigkeit ausgewählt ist aus Methylnaphthalinen, Xylol, Xylenolen, ortho-sec.-Butylphenol, para-Nonylphenol und Mischungen davon.

8. Ein Verfahren zir Bildung eines flüssigen Konzentrats, welches für die Markierung einer mit Wasser nicht mischbaren Flüssigkeit geeignet ist, umfassend den Schritt des Vermischens des folgenden:

A. von 100 000 TpM bis 300 000 TpM einer Markierungsverbindung mit einer Struktur ausgewählt aus

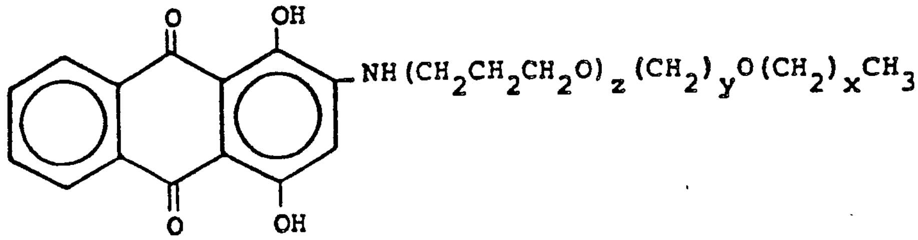

wobei x eine ganze Zahl von 0 bis einschließlich 3 ist, y eine ganze Zahl von 1 bis einschließlich 3 ist und z eine ganze Zahl von 0 bis einschließlich 1 bedeutet; und

B. von 700 000 bis 900 000 TpM einer als Verdünnungsmittel wirkenden Flüssigkeit, welche in der mit Wasser nicht mischbaren Flüssigkeit löslich ist.

9. Ein Verfahren zum Nachweis einer Markierungsverbindung mit einer Struktur ausgewählt aus:

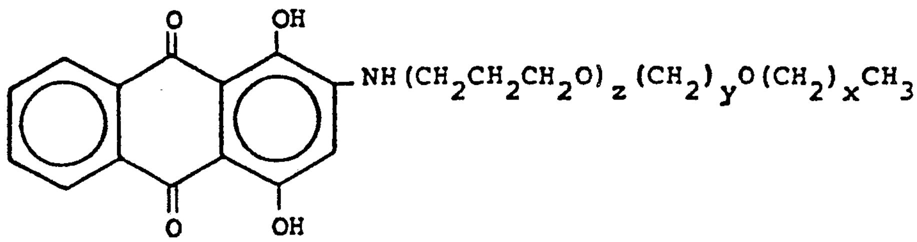

wobei x eine ganze Zahl von 0 bis einschließlich 3 ist, y eine ganze Zahl von 1 bis einschließlich 3 ist und z eine ganze Zahl von 0 bis einschließlich 1 bedeutet, und

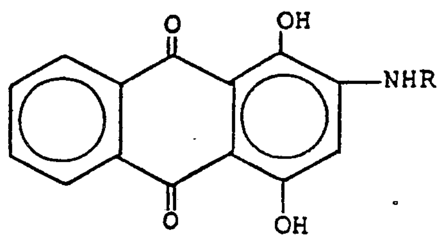

wobei R ausgewählt ist aus geradkettigen oder verzweigten Kohlenwasserstoffen mit 1 bis Kohlenstoffatomen, umfassend die folgenden Schritte:

A. Vorlegen einer Probe, von der angenommen wird, daß sie die Markierungsverbindung enthält und

a) Durchführen einer chemischen Reaktion durch Kontaktieren dieser Probe

B. mit einem Reagens enthaltend:

i) von 20 bis 60% Glycerin;

ii) von 20 bis 40% Wasser; und

iii) von 10 bis 50% eines organischen Amins;

C. Abtrennen des Reagens von der Probe; und

D. Bestimmen, ob das Reagens eine im wesentlichen bläulichrote Farbe annimmt, wodurch die Gegenwart der Markierungsverbindungen angezeigt wird, oder

b) Absorbieren der Markierungsverbindung und Konzentrieren derselben durch

B. Hindurchleiten der Probe durch einen Chromatographische Säule mit aktiviertem Aluminiumoxid;

C. Auswaschen der organischen Flüssigkeit aus der Säule;

D. Auswaschen der natürlichen Farben der organischen Flüssigkeit aus der Säule; und

E. Eluieren mit dem Reagens, enthaltend die Komponenten i), ii) und iii) wie unter aB beschrieben, wenn sich in der Säule eine im wesentlichen purpurfarbene Zone ausbildet, wodurch die Gegenwart der Markierungsverbindungen angezeigt wird.

**Revendications**

1. Liquide organique marqué non miscible à l'eau comprenant 1 ppm à 300 000 ppm d'un composé de marquage répondant à la formule:

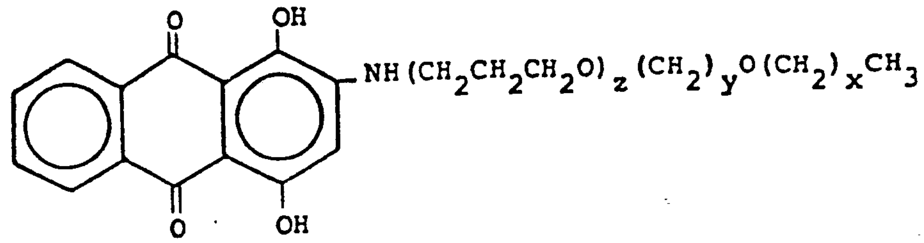

dans laquelle x est un nombre entier de 0 à 3 inclus, y est un nombre entier de 1 à 3 inclus et z est un nombre entier égal à 0 ou 1.

2. Liquide marqué suivant la revendication 1, comprenant 1 ppm à 15 ppm du composé de marquage.

3. Liquide marqué suivant la revendication 1, comprenant 10 ppm à 15 ppm du composé de marquage.

4. Liquide marqué suivant la revendication 1, sous forme d'un concentré comprenant 100 000 ppm à 300 000 ppm du composé de marquage.

5. Liquide marqué suivant la revendication 1, dans lequel ledit liquide est un combustible hydrocarboné.

6. Liquide marqué suivant les revendications 4 et 5, comprenant 700 000 à 900 000 ppm d'un liquide de dilution qui est soluble dans le liquide organique non miscible à l'eau.

7. Liquide marqué suivant la revendication 6, dans lequel le liquide de dilution est choisi entre des méthylnaphtalènes, le xylène, des xylénols, l'ortho-sec.-butylphénol, la para-nonylphénol et leurs mélanges.

8. Procédé de formation d'un concentré liquide utile pour le marquage d'un liquide non miscible à l'eau, comprenant l'étape consistant à mélanger ce qui suit:

A. 100 000 ppm à 300 000 ppm d'un composé de marquage répondant à la formule:

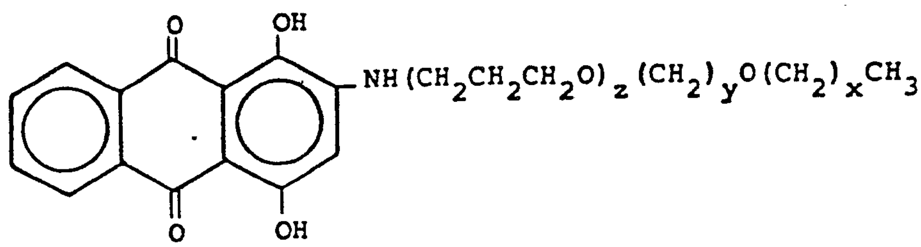

dans laquelle x est un nombre entier de 0 à 3 inclus, y est un nombre entier de 1 à 3 inclus et z est un nombre entier égal à 0 ou 1; et

B. 700 000 à 900 000 ppm d'un liquide de dilution, qui est soluble dans le liquide non miscible à l'eau.

9. Procédé de détection d'un composé de marquage choisi entre un composé de formule:

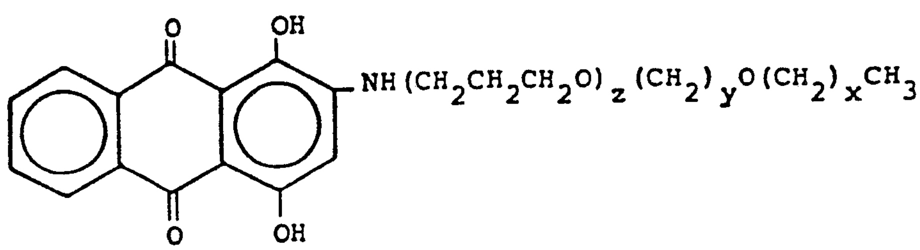

dans laquelle x est un nombre entier de 0 à 3 inclus, y est un nombre entier de 1 à 3 inclus et z est un nombre entier égal à 0 ou 1, et un composé de formule:

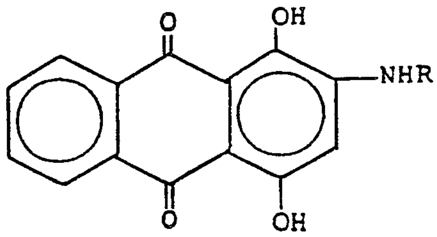

dans laquelle R est choisi entre des hydrocarbures linéaires ou ramifiés ayant 1 à 5 atomes de carbone, comprenant les étapes consistant:

A. à préparer un échantillon suspecté de contenir ledit composé de marquage et

a) à effectuer une réaction chimique par mise en contact dudit échantillon

B. avec un réactif comprenant:

i) 20% à 60% de glycérol;

ii) 20% à 40% d'eau; et

iii) 10% à 50% d'une amine organique;

C. à séparer ledit réactif et ledit échantillon; et

D. à déterminer si ledit réactif provoque l'apparition d'une couleur fortement rouge-bleuâtre, indiquant ainsi la présence desdits composés de marquage, ou

b) à absorber ledit composé de marquage et à le concentrer par un procédé consistant

B. à faire passer ledit échantillon à travers une colonne chromatographique d'alumine activée;

C. à enlever par lavage ledit liquide organique de ladite colonne;

D. à supprimer par lavage les couleurs naturelles dudit liquide organique de ladite colonne; et

E. à effectuer une élution avec le réactif comprenant les constituants i, ii et iii, tels qu'ils sont décrits en aB lorsqu'une zone de couleur fortement pourpre est formée dans ladite colonne, indiquant ainsi la présence desdits composés de marquage.